# EUROPÄISCHE PATENTSCHRIFT

(11) **EP 3 946 574 B1**
(45) Veröffentlichungstag und Bekanntmachung des Hinweises auf die Patenterteilung: **04.12.2024**
(21) Anmeldenummer: 20718807.9
(22) Anmeldetag: 02.04.2020
(51) Int. Cl.: A61N 5/06

(54) **BESTRAHLUNGSMODUL SOWIE VORRICHTUNG ZUM BESTRAHLEN MIT MEDIZINISCH-KOSMETISCHER STRAHLUNG**
IRRADIATION MODULE AND DEVICE FOR IRRADIATION WITH MEDICAL AND COSMETIC RADIATION
MODULE D'IRRADIATION AINSI QUE DISPOSITIF PERMETTANT L'IRRADIATION AVEC UN RAYONNEMENT MÉDICAL COSMÉTIQUE

(30) Priorität: 03.04.2019 CH 4562019
(43) Veröffentlichungstag der Anmeldung: 09.02.2022
(73) Patentinhaber: JK-Holding GmbH, 53578 Windhagen (DE)
(72) Erfinder: GERSTENMEIER, Jürgen, 56567 Neuwied (DE)
(74) Vertreter: IPrime Rentsch Kaelin AG
(86) Internationale Anmeldenummer: PCT/IB2020/053142
(87) Internationale Veröffentlichungsnummer: WO 2020/202060

(56) Entgegenhaltungen:
- EP-A1- 3 597 268
- RU-U1- 175 693
- US-A1- 2004 075 065
- US-A1- 2014 207 215
- US-A1- 2016 082 280

## Beschreibung

Die Erfindung betrifft ein Bestrahlungsmodul zum Einsatz in einer Vorrichtung zum Bestrahlen mit medizinisch-kosmetischer Strahlung, umfassend eine Mehrzahl von auf einem Träger angeordneter LEDs. Die Erfindung betrifft ferner eine Vorrichtung umfassend eine Mehrzahl von Bestrahlungsmodulen zum Bestrahlen mit medizinisch-kosmetischer Strahlung, die in einem Gehäuseteil untergebracht sind.

Aus der Praxis sind Vorrichtungen zum Bestrahlen mit medizinisch-kosmetischer Strahlung bekannt, die insbesondere als Solarium ausgebildet sind und bei denen ein Benutzer insbesondere mit ultravioletter Strahlung, insbesondere des Spektrums UV-A und des Spektrums UV-B, beaufschlagt wird, um eine Bräunung anzuregen. Die Bestrahlungsmodule sind hierbei als Niederdruck-Leuchtstoffröhren oder als Hochdrucklampen ausgebildet, die jedoch die Unterbringung in relativ großen Gehäuseteilen erforderlich machen und selbst einen recht großen Raum einnehmen. Darüber hinaus lassen sich die Leuchtstoffröhren und Hochdrucklampen kaum individuell auf das gewünschte Bestrahlungsergebnis einstellen, und insbesondere lassen sich die bekannten Vorrichtungen nicht auf unterschiedliche Benutzer optimal einstellen, sodass das Ergebnis der Bestrahlung bei schmalen oder kräftigen Personen unterschiedlich ausfällt. Schließlich verbrauchen die bekannten Vorrichtungen viel Energie.

Im Bereich der Bestrahlung der Haut werden medizinisch-kosmetische Strahlung emittierende Anordnungen eingesetzt, deren Strahlung eine photobiologische Wirkung bei einer bestrahlten Person erzeugt. Die medizinisch-kosmetische Strahlung trifft hierbei auf die Haut der Person auf, kann aber je nach konkreter Wellenlänge in tiefere Regionen des Körpers eindringen. Die Wirkung umfasst beispielsweise eine Bräunung der Haut, aber auch weitere physiologische und psychologische Effekte resultieren aus der Bestrahlung. Medizinisch-kosmetische Strahlung umfasst das Spektrum der ultravioletten (UV-) Strahlung, der sichtbaren (VIS-) Strahlung und der nahen Infrarot (nIR-) Strahlung. Die UV-Strahlung weist hierbei Wellenlängen im Spektrum zwischen 100 nm und ca. 380 nm auf, die VIS-Strahlung weist hierbei Wellenlängen im Spektrum zwischen ca. 380 nm und ca. 780 nm auf, die nIR-Strahlung weist hierbei Wellenlängen im Spektrum zwischen ca. 780 nm und 1400 nm auf. Die genannten Spektren gehen ineinander über. Je nach medizinisch-kosmetischer Anwendung kann die Bestrahlung auf ein Teilspektrum der genannten Spektren konzentriert sein. Hierzu können medizinisch-kosmetische Strahlung emittierende Anordnungen auch dediziert einzelnen Wellenlängen zugeordnet sein, z.B. der UV-Strahlung, die von Strahlröhren erzeugt wird. Jedoch ist der Einsatz einer Strahlröhre nicht zwingend. Wegen der therapeutischen Effekte der medizinisch-kosmetischen Strahlung kann sie auch als medizinisch-kosmetisch-therapeutische Strahlung bezeichnet werden. Das Spektrum der UV-A Strahlung reicht von etwa 380 nm bis 315 nm, das Spektrum der UV-B Strahlung reicht von etwa 315 nm bis 280 nm. Jenseits von 280 nm bis etwa 100 nm ist das Spektrum der UV-C Strahlung, die für den menschlichen Körper Schäden hervorrufen kann.

Aus der Praxis sind Vorrichtungen zur Einwirkung auf die Haut eines Benutzers bekannt, wie sie beispielsweise in Sonnenstudios eingesetzt werden, bei denen eine zu bestrahlende Person zwecks Bräunung ihrer Haut durch Pigmentierung auf einer eine Liegefläche bzw. Abschlussfläche bildenden Abdeckung liegen kann, wobei unterhalb der Abdeckung eine UV-Strahlung emittierende Anordnung mit in der Regel einer Mehrzahl von Strahlröhren, insbesondere Leuchtstoffröhren, angeordnet ist, wobei die Abdeckung für den Zugang zu den Strahlröhren abgenommen bzw. verschwenkt werden kann. Meist weisen solche Bräunungsgeräte auch eine weitere Baueinheit mit weiteren Strahlröhren und einer zweiten Abdeckung auf, die gemeinsam auf die zu bestrahlende Person geschwenkt werden können, sodass die Person von zwei Seiten gebräunt werden kann.

Aus der Praxis sind auch sogenannte Stehbräuner bekannt, bei denen die zu bestrahlende Person nicht horizontal auf der Abdeckung liegt, sondern in vertikaler Position von der UV-Strahlung emittierenden Anordnung umgeben ist. Bei einem Stehbräuner verlaufen die Strahlrohre insbesondere in vertikaler Richtung.

Vorrichtungen zur Einwirkung auf die Haut sind zum Beispiel aus US 2004/075065 A1 bekannt.

Es ist die Aufgabe der Erfindung, ein Bestrahlungsmodul bzw. eine Vorrichtung und ein Verfahren zum Bestrahlen anzugeben, mit denen jeweils ein verbessertes Bestrahlungsergebnis erreicht wird.

Die vorliegende Erfindung wird im Anspruch 1 definiert. Weitere vorteilhafte Ausführungsformen gehen aus den abhängigen Ansprüchen hervor.

Gemäß einem Aspekt der Erfindung ist ein Bestrahlungsmodul zum Einsatz in einer Vorrichtung zum Bestrahlen mit medizinisch-kosmetischer Strahlung geschaffen, umfassend eine Mehrzahl von auf einem Träger angeordneter LEDs wobei auf dem Träger eine erste Gruppe von LEDs, die Strahlung des Spektrums UV-A, und eine zweite Gruppe von zweiten LEDs, die Strahlung des Spektrums UV-B emittiert, angeordnet sind. Durch das gemischte Anordnen von LEDs der Spektren UV-A und UV-B wird ein günstiges Bestrahlungsergebnis erzielt, wobei insbesondere die LEDs des Spektrums UV-B einen sehr viel höheren photobiologischen Effekt aufweisen als die LEDs des Spektrums UV-A. Das Bestrahlungsmodul kann in der Vorrichtung zum Bestrahlen an verschiedenen Stellen angeordnet werden, beispielsweise im Bereich der Bestrahlung des Gesichts, im Bereich der Bestrahlung des Rumpfes und/oder im Bereich der Bestrahlung der Schultern.

Die ersten LEDs des Spektrums UV-A und die zweiten LEDs des Spektrum UV-B sind hierbei als unterschiedliche Chips ausgebildet, die auf dem Träger angeordnet werden, wobei sich die beiden LEDs auch in ihrer Abstrahlcharakteristik unterscheiden. Auch die Kontaktierung der LEDs ist hierbei unterschiedlich ausgeführt, wobei die LEDs der ersten Gruppe vorzugsweise gemeinsam angesteuert werden, und die LEDs der zweiten Gruppe ebenfalls gemeinsam angesteuert werden. Es ist aber möglich, die LEDs auch einzeln anzusteuern, oder aber mehrere erste Gruppen von ersten LEDs und mehrere zweite Gruppen von zweiten LEDs auf dem Träger vorzusehen.

Der Träger ist zweckmäßigerweise als flache Platte ausgebildet, die beispielsweise rechteckig ausgebildet ist. Hierdurch können Reflektoren mit einer zu dem Träger normalen Achse einfach nachgelagert angeordnet werden. Es ist alternativ auch möglich, den Träger bereits mit einer Krümmung auszubilden, sodass die LEDs auf einer konkaven Seite oder alternativ auf einer konvexen Seite des Trägers angeordnet werden, wodurch die Bestrahlungsmodule insgesamt eher in der Lage sind, der Geometrie eines menschlichen Körpers zu folgen. Es ist alternativ möglich, den Träger als gefaltetes Teil auszubilden, bei dem die LEDs auf den jeweils abgefalteten flachen Abschnitten zueinander einen Winkel einnehmen.

Der Träger kann auch recht große Abmessungen aufweisen, sodass zum Beispiel in einem Gehäuseteil einer Vorrichtung im Wesentlichen jeweils nur ein Träger angeordnet zu werden braucht. Je nach beabsichtigter Anwendung ist die Geometrie des Trägers frei gestaltbar. Eine besonders günstige Gestalt ergibt sich zum Beispiel dann, wenn der Träger ringsegmentförmig oder als Teil eines Polygons ausgebildet ist und damit eine Röhre für einen Benutzer zumindest teilweise umbaut. In diesem Fall sind die LEDs auf der Innenseite des Trägers angeordnet.

Die ersten LEDs machen zwischen 50% und 90% der Summe aus ersten und zweiten LEDs aus, und die zweiten LEDs machen zwischen 50% und 10% der Summe aus ersten und zweiten LEDs aus. Damit ist mindestens ein Zehntel und maximal die Hälfte der LEDs als zweite LEDs, die Strahlung des Spektrums UV-B emittieren, ausgebildet, was im Hinblick auf die deutlich höhere photobiologische Wirksamkeit zweckmäßig ist. Idealerweise beträgt der Anteil an ersten LEDs zwischen 70% und 80% und der Anteil an zweiten LEDs zwischen 20% und 30%.

Sind die ersten LEDs und die zweiten LEDs zu gleichen Teilen auf dem Träger vorgesehen, ist es besonders günstig, wenn diese sich nach Art eines Schachbrettmusters abwechseln. Alternativ können auch die Zeilen und/oder die Spalten abwechselnd mit ersten LEDs und zweiten LEDs ausgestattet sein.

Die Zahl der auf einem Träger angeordneten LEDs kann ohne Weiteres mehrere hundert LEDs betragen, was auch von der Größe des Trägers abhängt. Gemäß einer günstigen Ausgestaltung ist die Zahl der auf einem Träger angeordneten LEDs ausgewählt zwischen ungefähr 10 und ungefähr 600 LEDs, vorzugsweise jedoch zwischen 20 und 60 LEDs. Da von den LEDs eine spürbare Wärmeentwicklung ausgeht, ist es zweckmäßig, den Träger auf einer Wärmeübergangsplatte anzuschließen und diese mit externen Mitteln zu kühlen, sodass die Anzahl der zweckmäßigerweise auf einem Träger angeordneten LEDs, die mit einer Kühleinrichtung gekühlt werden, zum Beispiel 40 beträgt.

Vorzugsweise sind auf einem Träger zehn oder zwanzig LEDs oder ein ganzzahliges Vielfaches davon angeordnet, wobei jeweils zwei, vier oder ein ganzzahliges Vielfaches davon der LEDs als zweite LEDs ausgebildet sind. Hierdurch wird eine gute Mischung der erzeugten Strahlung der beiden Spektren der beiden Gruppen erreicht, wodurch sich eine günstige Bräunung eines Benutzers ergibt.

Zweckmäßigerweise sind die LEDs, die auf dem Träger angeordnet sind, ausschließlich solche der ersten Gruppe und der zweiten Gruppe, sodass sich die Summe der ersten LEDs und der zweiten LEDs zur Summe der UV-Strahlung emittierenden LEDs ergibt, und die zweiten LEDs 20% dieser Summe ausmachen.

Die LEDs sind in vorteilhafter Ausgestaltung auf dem Träger in einem Feld aus vier Zeilen und fünf Spalten angeordnet, wobei zweckmäßigerweise die mittlere Spalte durch zweite LEDs ausgebildet ist, während die übrigen Spalten durch erste LEDs ausgebildet sind. Hierdurch lässt sich ein geeignetes Verhältnis 80:20 beziehungsweise bei zwanzig LEDs von 16:4 erreichen, mit dem eine günstige Bestrahlungscharakteristik erreicht wird. Es ist möglich, mehrere der Bestrahlungsmodule nebeneinander anzuordnen und damit ganze Flächenbereiche mit Bestrahlungsmodulen auszugestalten.

Der Abstand benachbarter LEDs in derselben Spalte oder in derselben Zeile ist zweckmäßigerweise konstant und beträgt vorzugsweise zwischen 1 cm und 4 cm, besonders bevorzugt zwischen 1,25 cm und 2,25 cm und im Idealfall zwischen 1,5 cm und 2 cm. Hierdurch können etwa vorzusehende Reflektoren noch ausreichend groß bemessen sein, um die von den LEDs ausgehende Strahlung zu vergleichmäßigen und damit ein gleichmäßiges Bestrahlungsergebnis zu erreichen.

Zweckmäßigerweise sind die ersten LEDs und/oder die zweiten LEDs mit einer Primäroptik in Gestalt einer Silikonlinse ausgestattet, die bewirkt, dass die Abstrahlung des LEDs im Wesentlichen entsprechend der gewünschten Richtung austritt. Die Primäroptik bündelt die von den LEDs ausgesandte Strahlung und verhindert damit unnötige Verluste. Statt einer Silikonlinse kann die Primäroptik auch in anderer Weise verwirklicht sein.

Vorzugsweise werden die zweiten LEDs mit einer geringeren Leistung angesteuert als die ersten LEDs, da der photobiologische Effekt der zweiten LEDs trotz des geringeren Wirkungsgrads der zweiten LEDs wesentlich höher ausfällt. Hierdurch sollen insbesondere Verbrennungen bei einer zu bestrahlenden Person wirksam unterbunden werden.

Der Träger ist an eine Wärmeübergangsplatte angeschlossen, um die durch den Betrieb der LEDs entstehende Wärme ableiten zu können. Die Wärmeübergangsplatte ist hierbei zweckmäßigerweise über Kühlleitungen mit einem Wärmetauscher verbunden, der je nach Einbausituation auch beabstandet von der Wärmeübergangsplatte angeordnet sein kann. Die Wärmeübergangsplatte weist hierzu zweckmäßig Kanäle oder Kapillaren auf, in denen ein zum Wärmetauscher über die Kühlleitungen geführtes Fluid zirkulieren und die Platte kühlen kann. Das Fluid kann ein Gas, ein Gas-Flüssigkeitsgemisch oder eine Flüssigkeit sein, wobei darüber hinaus Lüftungsschlitze in der Wärmeübergangsplatte angeordnet sein können, die zu einer Kühlung mit Umgebungsluft führen.

Jeder LED ist ein Reflektor zugeordnet, der an den Träger angeschlossen ist, und durch den eine gleichmäßigere Abgabe der Strahlung der jeweiligen LED erreicht wird. Der Reflektor ist besonders dann vorteilhaft, wenn die gleichmäßige Verteilung der Strahlung nicht von der Primäroptik erzielt werden kann.

Die Reflektoren erweitern sich von den LEDs aus kegelig fort und sind in einer gemeinsamen Baueinheit angeordnet, die es ermöglicht, die gesamte Baueinheit mit dem Träger zu verbinden. Hierbei sind die Achsen der Reflektoren zweckmäßig auf die Abstände der LEDs abgestimmt, sodass die Baueinheit auf dem Träger jeweils für alle LEDs zentriert werden kann. Hierbei ist zweckmäßigerweise vorgesehen, dass die ersten LEDs und die zweiten LEDs mit demselben Reflektor ausgestattet sind, sodass die ersten LEDs und zweiten LEDs an unterschiedlichen Positionen angeordnet sein können, ohne dass hierfür die Baueinheit verschieden ausgebildet sein muss.

Die Baueinheit mit den Reflektoren, der Träger und die Wärmeübergangsplatte sind miteinander verbunden, sodass diese gemeinsam in einer Vorrichtung zur Bestrahlung eingesetzt werden können. Die Verbindung erfolgt vorzugsweise durch Verschraubung, es ist aber auch möglich, die genannten Teile miteinander zu vernieten, zu verkleben oder in anderer Weise miteinander zu verbinden.

Gemäß einer besonders bevorzugten Weiterbildung ist vorgesehen, dass die Baueinheit für die Reflektoren wenigstens eine Aussparung für eine ohne Reflektor ausgestattete dritte LED aufweist, die sichtbare Strahlung emittiert. Die als beispielsweise rotes Licht aussendende LED kann hierzu in einem Bereich, der von vier Reflektoren umgeben ist, angeordnet werden, sodass die dritte LED, die sichtbare Strahlung aussendet, für die gleichzeitige oder sukzessive Anwendung einer Lichttherapie in der Vorrichtung zum Bestrahlen genutzt werden kann.

Wenn das Bestrahlungsmodul ohne Reflektoren ausgestattet ist, kann die dritte LED, die sichtbare Strahlung emittiert, auch auf dem Träger positioniert sein. Die dritten LEDs sind bei der Ermittlung des Verhältnisses der ersten LEDs und zweiten LEDs nicht zu berücksichtigen. Es ist alternativ auch möglich, die dritte LED mit einem Reflektor auszustatten, bei dem es sich um denselben Reflektor wie die ersten und/oder zweiten LEDs handeln kann, aber nicht muss.

In einer bevorzugten Ausgestaltung ist vorgesehen, dass die Wärmeübergangsplatte über eine Steckverbindung mit den Kühlleitungen verbunden ist. Hierdurch kann die Gruppe aus Wärmeübergangsplatte, Träger und Baueinheit von Reflektoren in leichter Weise ausgetauscht werden, auch innerhalb einer Vorrichtung zur Bestrahlung, und je nach Anwendungsfall ein Träger mit unterschiedlicher Bestückung von ersten LEDs und zweiten LEDs eingesetzt werden. Darüber hinaus kann ein Träger dann, wenn die LEDs beschädigt sind oder von ihrer Charakteristik her nicht sinnvoll einsetzbar sind, in einfacher Weise ausgetauscht werden.

In weiterer vorteilhafter Ausgestaltung kann zugleich eine der Kühlleitungen als elektrischer Leiter zur Kontaktierung der auf dem Träger angeordneten LEDs ausgebildet sein, sodass neben dem Austausch des Trägers zugleich auch die elektrische Kontaktierung nach Art einer Steckerlösung verwirklicht ist.

In günstiger Weiterbildung ist die Baueinheit mit den Reflektoren mit einer Abdeckscheibe ausgestattet, die eine der Anzahl von Reflektoren entsprechende Anzahl von kreisförmigen Ausnehmungen aufweist, wobei die Scheibe vorzugsweise aus Acrylglas ausgebildet ist. Die kreisförmigen Aussparungen sind hierbei zu den Achsen der Reflektoren fluchtend ausgebildet und an ihrem Innenumfang mit einem fluoreszierenden Belag ausgestattet, der auf Grund der Anregung durch die von den ersten LEDs und/oder zweiten LEDs emittierten Strahlung zum Leuchten angeregt wird. Auf diese Weise wird vorteilhaft erreicht, dass ein Benutzer, der von einem Bestrahlungsmodul mit Strahlung beaufschlagt wird, unterscheiden kann, ob die nicht im sichtbaren Bereich strahlenden ersten LEDs und/oder zweiten LEDs aktiv eine Strahlung aussenden oder nicht. Die Scheibe wird zweckmäßigerweise gemeinsam mit der Baugruppe aus Wärmeübergangsplatte, Träger und Baueinheit verschraubt, sodass vorzugsweise im Falle des Vorsehens von dritten LEDs in der Scheibe auch Bohrungen für den Durchtritt der von diesen emittierten sichtbaren Strahlung vorgesehen sind. Es ist möglich, die fluoreszierende Beschichtung so auszuwählen, dass sie in einer Farbe leuchtet, die einen fototherapeutischen Effekt auf den menschlichen Körper hat.

Eine günstige Verwendung eines Bestrahlungsmoduls ist in einer Einheit zum Bräunen der Schultern gegeben. Eine weitere günstige Verwendung eines Bestrahlungsmoduls ist in einer Einheit zum Bräunen des Gesichts gegeben. Eine weitere günstige Verwendung eines Bestrahlungsmoduls ist in einer Vorrichtung zum Bräunen des ganzen Körpers, insbesondere des Rumpfes, der Arme und der Beine eines Benutzers gegeben.

Gemäß einem Aspekt der Erfindung zeichnet sich eine Vorrichtung zum Bestrahlen mit medizinisch-kosmetischer Strahlung dadurch aus, dass wenigsten ein Bestrahlungsmodul wie vorstehend beschrieben darin vorgesehen ist. Es ist möglich, eine Mehrzahl von Bestrahlungsmodulen in der Vorrichtung anzuordnen, die nebeneinander einen Tunnel oder eine Röhre umschließen, in der ein Benutzer stehend oder liegend Platz finden kann, um unter der Einwirkung der Strahlung insbesondere seine Haut zu bräunen.

Die Bestrahlungsmodule sind vorteilhaft von geringen Abmessungen und geringem Gewicht, so dass sie sich leicht einbauen und/oder auswechseln lassen. Die Vorrichtung sieht dann vorteilhaft Mittel zum elektrischen Anschluss der Bestrahlungsmodule vor, die sich je nach beabsichtigter Anwendung einsetzen und anschließen oder auch wieder abklemmen oder herausnehmen lassen.

Die Vorrichtung weist zweckmäßigerweise eine Reihe von Steckplätzen auf, an denen beispielsweise die vorstehend beschriebenen Bestrahlungsmodule, aber auch andere Module wie solche mit dritten LEDs aus dem sichtbaren Spektrum angeschlossen werden können. Auf dieses Weise kann die Vorrichtung wahlweise als Bräunungseinrichtung betrieben werden, wenn die vorgenannten Bestrahlungsmodule oder andere Bestrahlungsmodule eingesetzt werden, oder als Lichttherapieeinrichtung, wenn Module mit dritten LEDs aus dem sichtbaren Spektrum angeschlossen werden. Zugleich kann die Vorrichtung durch die Modularität einfach benutzerspezifisch umgebaut werden. Hierzu ist vorteilhaft vorgesehen, dass eine Steuerung die eingesetzten Module erkennt und sicherstellt, dass sie in zulässiger Weise betrieben werden. Auf diese Weise lässt sich ein defektes Modul leicht austauschen und ersetzen.

Gemäß einem Aspekt der Erfindung ist eine Vorrichtung zum Bestrahlen mit medizinisch-kosmetischer Strahlung geschaffen, umfassend eine Mehrzahl von Bestrahlungsmodulen zum Bestrahlen mit medizinisch-kosmetischer Strahlung, die in einem Gehäuseteil untergebracht sind, wobei zumindest eines der Bestrahlungsmodule einzeln oder gemeinsam mit anderen Bestrahlungsmodulen innerhalb des Gehäuseteils in Richtung auf einen Benutzer zustellbar ist. Hierdurch wird vorteilhaft erreicht, dass die Bestrahlungsmodule und damit die Vorrichtung ein körperkonturnahes und damit im Energieverbrauch günstiges Bestrahlen eines in der Vorrichtung angeordneten Benutzers möglich ist, wobei insbesondere eine Anpassung an die Körpergeometrie des Benutzers möglich ist. Bei großen oder kräftig gebauten Benutzern wird das Bestrahlungsmodul zurückgezogen, um Verbrennungen auf der Haut oder ein Überschreiten der Bestrahlungsdosis zu vermeiden. Bei kleinen oder schmal gebauten Benutzern wird das Bestrahlungsmodul vorgeschoben, um ein optimales Bestrahlungsergebnis zu erreichen. Neben der im Wesentlichen axialen Verstellbarkeit in Richtung auf den Körper des Benutzers ist es auch möglich, das Bestrahlungsmodul schwenkbar anzuordnen und damit hin und her zu bewegen, wodurch eine Vergleichmäßigung des Bestrahlungsergebnisses erreichbar ist. Zugleich können von dem Bestrahlungsmodulen schwer erreichbare Zonen wie beispielsweise Schulter oder Körperhöhlungen unter verschiedenen Winkeln bestrahlt und damit gebräunt werden.

Die Zustellung der Bestrahlungsmodule erfolgt hierbei wahlweise pneumatisch, hydraulisch oder mechanisch, z.B. mittels einer Zahnstange oder eines Spindel-/Spindelmutter-Systems, wobei zweckmäßigerweise der Antrieb mit einem Elektromotor vorgesehen ist, da die Vorrichtung ohnehin mit elektrischer Energie betrieben wird. Sollen mehrere Bestrahlungsmodule an den Körper des Benutzers herangeführt werden, ist zweckmäßigerweise vorgesehen, dass die Übergänge zwischen benachbarten Modulen mittels einer Abdichtung, beispielsweise eines Membranüberzugs, abgedichtet sind, um Schwierigkeiten bei der Reinigung zu vermeiden. Es ist aber auch möglich, ganze Abschnitte des Gehäuseteils insgesamt zu verschieben, nachdem die Röhre oder der Tunnel gebildet ist, wobei das Gehäuseteil dann vergleichsweise körpernah angeordnet ist. Die Bestrahlungsmodule werden dann innerhalb des Gehäuseteils in Richtung auf den Körper des Benutzers zugestellt, um durch die Optimierung der Entfernung ein günstiges Ergebnis der Bestrahlung bei geringem Energieverbrauch zu erreichen.

Um eine dreidimensionale Zustellung der Bestrahlungsmodule günstig verwirklichen zu können, ist zweckmäßigerweise das Bestrahlungsmodul mit einem wabenförmigen Träger ausgebildet, der beispielsweise eine hexagonale Fläche ausbildet, wobei an jeder Kante jeweils ein benachbarter Wabenkörper angeschlossen ist. Alternativ kann mit den hexagonalen Trägern auch ein Träger mit anderer Geometrie vorgesehen sein, um eine sphärische Krümmung zu erreichen.

Gemäß einer anderen alternativen Ausgestaltung können die Träger mit den LEDs auch auf einer dreidimensionalen Fläche, die eine flexible Zustellung zulässt, angebracht sein, wodurch nicht das einzelne Bestrahlungsmodul, sondern die Fläche insgesamt verlagerbar ausgebildet ist.

Gemäß einer vorteilhaften Ausgestaltung ist vorgesehen, dass Mittel zum Messen des Abstandes des Benutzers zu dem zumindest einem Bestrahlungsmodul vorgesehen sind, die zweckmäßigerweise in dem Gehäuseteil angeordnet sind, sodass der Abstand des Benutzers zu dem Gehäuseteil in einem charakteristischen Punkt oder in mehreren Punkten, die einen Körperverlauf ergeben, möglich ist. Die Mittel zum Messen des Abstandes des Benutzers können optische Mittel sein, es ist aber auch möglich, an einem Gehäuseteil einen Maßstab wie beispielsweise ein Schachbrettmuster vorzusehen, das die Vermessung des Körpers des Benutzers und damit die Ermittlung des Abstandes zu den jeweiligen Bestrahlungsmodulen ermöglicht. Der Maßstab kann auch in einem von dem Gehäuseteil reflektierten Bild durch die Messmittel erfasst werden.

Das Bestrahlungsmodul ist dann zweckmäßigerweise derart in Richtung auf den Benutzer zustellbar, dass das Bestrahlungsmodul stets einen voreingestellten, optimalen Abstand zu dem Benutzer einhält. Dieser Abstand, der auch aus Sicherheitsgründen vorgegeben sein kann, ermöglicht ein besonders günstiges Ergebnis der Bestrahlung bei zugleich Einhaltung von Grenzwerten der Bestrahlung, bei zugleich möglichst geringem Energieverbrauch. Die Bestrahlungsmodule werden hierbei zweckmäßigerweise mit einer voreingestellten Leistung betrieben, und das Bestrahlungsergebnis ist hierbei im Wesentlichen auf Grund der Zustellung auf den Körper des Benutzers optimiert.

Zweckmäßigerweise werden benachbarte Bestrahlungsmodule durch eine flexible Membran gegen Eindringen von Verunreinigungen abgedichtet. Gerade dann, wenn die einzelnen Bestrahlungsmodule unterschiedlich weit vorgeschoben werden, besteht die Gefahr der Spaltbildung zwischen den benachbarten Modulen, in die Verunreinigungen eindringen können. Hierzu ist es erforderlich, eine Abdichtung auszuwählen, die auch gegen medizinisch-kosmetische Strahlung, insbesondere deren UV-Anteil, resistent ist.

Vorzugsweise ist jedoch vorgesehen, dass die Verlagerung der Bestrahlungsmodule innerhalb des Gehäuseteils erfolgt, sodass die Abdichtungen durch das aus Acrylglas gebildete Gehäuseteil ermöglicht ist.

Die Bestrahlungsmodule sind vorzugsweise rechteckig ausgebildet, insbesondere quadratisch. Damit lassen sich Flächen aus einer Mehrzahl von Bestrahlungsmodulen auf einfache Weise bilden. Alternativ können die Bestrahlungsmodule auch wabenförmig oder kreisförmig ausgebildet sein, oder aber eine Sphäre aufweisen, die es erlaubt, die Strahlung besonders effizient in Richtung auf den Benutzer zu richten.

Gemäß einem Aspekt der Erfindung ist eine Vorrichtung zum Bestrahlen eines Benutzers mit medizinisch-kosmetischer Strahlung geschaffen, umfassend eine Mehrzahl von Bestrahlungsmodulen zum Bestrahlen mit medizinisch-kosmetischer Strahlung, die in einen Gehäuseteil untergebracht sind, wobei die Bestrahlungsmodule eine Mehrzahl von LEDs aufweisen, die Strahlung des Spektrums UV-A und/oder des Spektrums UV-B emittieren, wobei die LEDs einzeln oder gemeinsam, insbesondere in Gruppen von LEDs mit demselben Spektrum, ansteuerbar sind, um mit einer vorgebbaren Intensität abzustrahlen. Hierdurch wird vorteilhaft eine Vorrichtung geschaffen, die die Bestrahlung des Benutzers mit medizinisch-kosmetischer Strahlung punktuell oder flächig ermöglicht, indem nur die LEDs eingesetzt werden, die für eine gewünschte Bestrahlung benötigt werden. Hierdurch lässt sich nicht nur Energie einsparen und die Belastung des Körpers des Benutzers schonen, sondern darüber hinaus können durch die einzelne oder gruppenweise Ansteuerung der LEDs auch bestimmte Programme für die Bestrahlung eingestellt werden, beispielsweise die LEDs gepulst eingesetzt werden, oder je nach Körperregion eine Beaufschlagung mit unterschiedlichen Strahlungsintensitäten erreicht werden.

Gemäß einer bevorzugten Ausgestaltung ist vorgesehen, dass eine von einem Benutzer belegte Fläche der Vorrichtung erfasst wird, und dass die der nicht belegten Fläche zugeordneten LEDs wahlweise mit verminderter Leistung oder gar nicht angesteuert werden. So kann beispielweise in Reaktion auf die variierende Körpergröße die LED bzw. das Bestrahlungsmodul, das dem Fußbereich zugeordnet ist, angesteuert werden oder abgeschaltet bleiben. Bei einer großen Person ist die Bestrahlungsleistung im Fußbereich erforderlich, bei einer weniger großen Person ist diese Strahlung ohne Nutzen. Hierdurch wird zweckmäßig Energie eingespart und auch die Wärmeentwicklung in der Vorrichtung reduziert. In entsprechender Weise kann das Ansteuern der LEDs beziehungsweise der Bestrahlungsmodule bei unterschiedlich breiten Personen erfolgen.

In besonders zweckmäßiger Weiterbildung ist vorgesehen, dass die Vorrichtung eine Eingabeeinheit aufweist, insbesondere ein Touch-Display, über das der Benutzer Körperzonen, die mit reduzierter Intensität bestrahlt werden sollen, auswählen oder definieren kann, und dass die Steuerung in Reaktion hierauf die Leistung der LEDs bzw. Bestrahlungsmodule reduziert. Insbesondere mit den punktförmig ausgestalteten LEDs können die Bereiche scharf umrissen werden, was bei als Leuchtstoffröhren ausgebildeten Bestrahlungsmodulen schwierig ist. Darüber hinaus ist die Ansteuerung der LEDs mit einer vergleichsweise linearen Charakteristik möglich, sodass auch ein nahezu stufenloses Einstellen der Bestrahlungsintensität des LEDs möglich ist.

Gemäß einem Aspekt der Erfindung ist eine Vorrichtung zum Bestrahlen eines Benutzers mit medizinisch-kosmetischer Strahlung geschaffen, umfassend eine Mehrzahl von Bestrahlungsmodulen zum Bestrahlen mit medizinisch-kosmetischer Strahlung, die in einem Gehäuseteil untergebracht sind, wobei Mittel zum Erfassen des zu bestrahlenden Benutzers vorgesehen sind, und wobei die Bestrahlungsmodule oder einzelne Strahlungsquellen der Bestrahlungsmodule in Abhängigkeit von Charakteristika des erfassten Benutzers ansteuerbar sind. Hierdurch wird vorteilhaft eine Vorrichtung geschaffen, die Lage und Beschaffenheit des Körpers des Benutzers charakterisiert und die Strahlenleistung der Bestrahlungsmodule auf Lage und/oder Beschaffenheit, insbesondere Dimensionen, des Körpers abstimmt. Hierdurch können vorteilhaft zum einen möglicherweise nicht benötigte Strahlungsquellen mit verminderter oder ohne Leistung betrieben werden, wodurch der Energieverbrauch gesenkt wird. Überdies können die Strahlungsmodule in ihrer Entfernung zu dem Körper optimal eingestellt werden, in der Regel in einen Abstand von circa 20 cm bis 30 cm zu der Körperoberfläche. Je nach Gestalt, zum Beispiel dick oder dünn, des Körpers des Benutzers werden die Bestrahlungsmodule mit der erforderlichen Intensität und/oder mit dem erforderlichen Abstand der Bestrahlungsmodule zu dem Körper betrieben, sodass für jeden Benutzer eine individuell optimierte Bestrahlung erreicht wird. Im Unterschied zu der aus dem Stand der Technik bekannten Lösung, bei der die Bestrahlungsmodule auf eine umhüllende Sphäre des Körpers ausgelegt sind, die der Körper nicht überschreitet, um gesundheitsschädigende Bestrahlungen zu vermeiden, ermöglicht die Vorrichtung ein individuelles Anpassen an den Körper, sodass insbesondere schlanke Personen, die einen großen Abstand von der gedachten Umhüllenden aufweisen, mit ausreichender Bestrahlungsdosis versorgt werden.

Zweckmäßigerweise ist zum Erfassen eines zu bestrahlenden Benutzers ein Sensor vorgesehen, der den Körper des Benutzers erfasst. Dies kann in einfacher Ausführungsform eine Kamera sein, die ein Bild der Vorrichtung mit und ohne Benutzer miteinander vergleicht und ausgehend hiervon bestimmt, welche Bestrahlungsmodule bzw. LEDs nicht benötigt werden. Die Kamera kann als CCD-Zeilenkamera ausgebildet sein, um zu vermeiden, dass Bilder der Benutzer auf missbräuchliche Weise erstellt werden. Alternativ kann die Kamera auch insbesondere für die von den Beleuchtungsmodulen emittierte Strahlung ausgelegt sein, sodass diejenigen Beleuchtungsmodule bzw. LEDs, die nicht von dem Körper des Benutzers abgeschattet werden, von der Kamera erfasst werden. Um die einzelnen Module bzw. LEDs besser identifizieren zu können, können diese mit bestimmten Frequenzen angesteuert werden, die eine eindeutige Zuordnung des Bestrahlungsmoduls bzw. der LED zu einem bestimmten Ort durch die in der Steuerung vorgesehenen Auswerteschaltung der Kamera ermöglichen.

Gemäß einem Aspekt der Erfindung ist eine Vorrichtung zum Bestrahlen eines Benutzers mit medizinisch-kosmetischer Strahlung geschaffen, umfassend eine Mehrzahl von Bestrahlungsmodulen zum Bestrahlen mit medizinisch-kosmetischer Strahlung, die in einem Gehäuseteil untergebracht sind, wobei ein Sensor vorgesehen ist, der die von den Bestrahlungsmodulen emittierte Strahlung erfasst, und wobei die Bestrahlungsmodule in Reaktion auf eine Abweichung der erfassten Strahlung von einem vorgebbaren Wert der Strahlung von einer Steuerung mit veränderten Betriebsparametern ansteuerbar sind, um die emittierte Strahlung an den vorgebbaren Wert anzupassen. Hierdurch wird vorteilhaft eine Vorrichtung geschaffen, die dynamisch eine Anpassung der Bestrahlungsmodule an eine vorgegebene Strahlungsleistung ermöglicht. Zum einen wird hierdurch vermieden, dass durch möglicherweise zu stark eingestellte Bestrahlungsmodule die maximal zulässige Strahlungsintensität für einen Benutzer überschritten wird. Eine solche zu hohe Strahlungsintensität kann auch dadurch eintreten, dass einzelne Strahlungsquellen der Bestrahlungsmodule, insbesondere LEDs, ihre Strahlungscharakteristik im Laufe der Betriebsdauer verändern, und untypisch nicht mit geringerer Intensität abstrahlen, sondern mit zunehmender Intensität. Die damit einhergehende Überschreitung der Strahlendosis für einen Benutzer wird durch den Sensor zuverlässig detektiert und durch Anpassen der emittierten Strahlung nach unten auf den vorgebbaren Wert vermieden. Umgekehrt kann es sein, dass durch Alterung oder durch andere Effekte ein Bestrahlungsmodul den vorgebbaren Wert der Strahlung nicht erreicht. In diesem Fall ermöglicht die Steuerung eine Kompensation der Strahlung mit der Folge, dass das gewünschte Bestrahlungsergebnis jederzeit zuverlässig gewährleistet ist. Im Unterschied zu den Lösungen aus dem Stand der Technik, bei denen eine Korrektur der Ansteuerung der Bestrahlungsmodule über experimentell ermittelte Kennkurven der Strahlungscharakteristik korrigiert werden, ist die Regelung dynamisch und praktisch vor oder nach jedem Bestrahlungsvorgang und sogar während des Bestrahlungsvorgangs möglich. Hierdurch können insbesondere Abweichungen, die im Laufe des Tages auftreten, vorteilhaft ausgeglichen werden. So ist beispielsweise bei manchen Vorrichtungen beobachtet worden, dass die Strahlungsintensität nach dem Anfahren der Vorrichtung nach einer längeren Unterbrechung über einen ersten Zeitraum geringer ist als die in der Folge abgegebene durchschnittliche Strahlungsintensität. Zugleich ist festgestellt worden, dass die Strahlungsintensität auch von der Umgebungstemperatur der Vorrichtung abhängt, sodass sich unterschiedliche Werte je nach Klimatisierung der Vorrichtung ergeben. Schließlich ist festgestellt worden, dass die empirisch ermittelten Kennkurven nur eine ungefähre Anpassung auf Leistungsverluste durch Alterung ermöglichen, und dass die Streuung so groß ist, dass individuell zu hohe oder zu niedrige Bestrahlungsdosen erzeugt werden. Insbesondere in der Praxis besteht ein Problem darin, dass teilweise nicht alle Bestrahlungsmodule ausgetauscht werden, sondern nur einige, beispielsweise Strahlröhren, die bereits optisch wahrnehmbar flackern. Richtet sich die Steuerung nun an der Kennkurve der neuen Strahlröhren aus, ist die Leistung der verbliebenen alten Leuchtröhren zu schwach, und umgekehrt. Hier ermöglich die dynamische Regelung der Strahlungsintensität auch bei einer solchen Mischbestückung mit Bestrahlungsmodulen ein dem vorgebbaren Wert der Strahlung entsprechendes Ergebnis.

Gemäß einer ersten bevorzugten Ausgestaltung ist vorgesehen, dass die Bestrahlungsmodule mit konstanter Spannung betrieben werden, und dann die Ansteuerung der Bestrahlungsmodule mittels Pulsweitenmodulation erfolgt. Hierdurch kann bei erfassten Abweichungen die Pulsweite angepasst und die Strahlungsintensität der Bestrahlungsmodule eingestellt werden.

Gemäß einer anderen bevorzugten Ausgestaltung ist vorgesehen, dass die Bestrahlungsmodule mit konstantem Strom betrieben werden, und dass die Ansteuerung der Bestrahlungsmodule mittels Veränderung der Stromstärke erfolgt. Hierdurch kann kontinuierlich die Strahlungsintensität hin auf den vorgebbaren Wert verändert werden.

Die Merkmale der vorstehend beschriebenen Vorrichtungen und Bestrahlungsmodule können ohne Weiteres auch in einer gemeinsamen Vorrichtung kombiniert werden, sodass insbesondere auch eine Vorrichtung, die mehrere der vorgenannten Merkmale miteinander kombiniert, Gegenstand der vorliegenden Offenbarung ist.

Ein Verfahren zum Bestrahlen mit medizinisch-kosmetischer Strahlung, insbesondere in einer Vorrichtung wie vorstehend beschrieben , wird zu Illustrationszwecken beschrieben, umfassend eine Mehrzahl von Bestrahlungsmodulen zum Bestrahlen mit medizinisch-kosmetischer Strahlung, die ausgewählt sind aus der Gruppe umfassend Leuchtstoffröhren, LEDs, organische LEDs und Hochdrucklampen, wobei die Bestrahlungsmodule wahlweise nur ein Teilspektrum der medizinisch-kosmetischen Strahlung emittieren, insbesondere das vorstehend beschriebene Teilspektrum für UV-A Strahlung und/oder das vorstehend beschriebene Teilspektrum für UV-B Strahlung. Hierbei ist ein Sensor vorgesehen, der die von dem Bestrahlungsmodul emittierte Strahlung erfasst, und die Bestrahlungsmodule werden in Reaktion auf eine Abweichung der erfassten Strahlung von einem vorgebbaren Wert der Strahlung von einer Steuerung mit veränderten Betriebsparametern angesteuert, um die emittierte Strahlung an den vorgebbaren Wert anzupassen. Hierdurch wird vorteilhaft eine einfache und zuverlässige Regelung der Strahlungsintensität einzelner Strahlungsquellen, einzelner Strahlungsmodule oder der gesamten Vorrichtung erreicht, sodass sichergestellt ist, dass der Benutzer keiner höheren Bestrahlungsdosis ausgesetzt ist als gewünscht oder zulässig, zugleich aber auch sichergestellt ist, dass die Bestrahlungsdosis ausreichend bemessen ist, um den Effekt der Bestrahlung, in der Regel eine Bräunung der Haut, zu erreichen.

Zweckmäßigerweise weist der Sensor eine hohe Empfindlichkeit für Strahlung im UV-Spektrum auf, die anders als sichtbare Strahlung auch eine Gefahrenquelle bei überlanger Exposition des menschlichen Körpers darstellt. Hierdurch wird zuverlässig vermieden, dass insbesondere die mit der Gefahr von Verbrennungen einhergehenden Spektren der medizinisch-kosmetischen Strahlung falsch eingestellt werden.

Zweckmäßigerweise ist wenigstens ein weiterer Sensor vorgesehen, der wahlweise dasselbe Spektrum oder ein hiervon verschiedenes Spektrum wie der erste Sensor erfasst.

Vorzugsweise ist vorgesehen, dass einem oder mehreren der Bestrahlungsmodule jeweils ein eigener Sensor zugeordnet ist, und dass die Bestrahlungsmodule auch einzeln an- und abschaltbar sind, um spezifische Strahlungsintensitäten für jedes Modul durch den Sensor erfassen zu können. Es ist möglich, beispielsweise eine einzelne LED auf einem LEDs umfassenden Bestrahlungsmodul in Hinblick auf die Strahlungsintensität einzustellen. Vorzugsweise wird jedoch das gesamte Bestrahlungsmodul eingestellt, was auf Grund der gleichartigen verwendeten LEDs in der Regel ausreichend ist, um eine Abweichung von den vorgebbaren Strahlungswerten zu vermeiden.

Es ist möglich, die Bestrahlungsmodule bzw. die LEDs der Bestrahlungsmodule in einzelnen Segmenten, die Körperbereichen entsprechen, anzusteuern, um das Bestrahlungsergebnis, in der Regel eine Bräunung der Haut, entsprechend der Empfindlichkeit der Haut einzustellen. Es ist möglich, die Empfindlichkeit der Haut mit einem spezifischen Detektor vor dem Bräunen oder während der Bräunung zu erfassen.

Es ist möglich, eine Mehrzahl von Sensoren in der Vorrichtung vorzusehen, die jeweils einen Bereich, der einem Körperbereich des Benutzers entspricht, zuverlässig überwachen zu können.

Ein Verfahren zum Bestrahlen eines Benutzers mit medizinisch-kosmetischer Strahlung wird zu Illustrationszwecken beschrieben, insbesondere wie vorstehend beschrieben, umfassend eine Mehrzahl von Bestrahlungsmodulen zum Bestrahlen mit medizinisch-kosmetischer Strahlung, die ausgewählt sind aus der Gruppe umfassend Leuchtstoffröhren, LEDs, organische LED und Hochdrucklampen, wobei die Bestrahlungsmodule wahlweise nur ein Teilspektrum der medizinisch-kosmetischen Strahlung emittieren, bei dem ein Benutzersensor vorgesehen ist, der Körpereigenschaften des Benutzers erfasst, und bei dem die Bestrahlungsmodule in Reaktion auf von dem Benutzersensor erfasste Körpereigenschaften von einer Steuerung mit Betriebsparametern ansteuerbar sind, die die emittierte Strahlung an erfasste Körpereigenschaften anpassen. Hierdurch ist es vorteilhaft möglich, benutzerspezifische Parameter durch einen Benutzersensor zu erfassen, und in Reaktion auf die erfassten Daten die Vorrichtung und insbesondere die Bestrahlungsmodule auf den Benutzer und seine Körpereigenschaften einzustellen. Hierdurch wird vorteilhaft die Bestrahlung effektiv gestaltet und sichergestellt, dass für den Benutzer schädliche Einstellungen der Vorrichtung vermieden werden. Der Benutzersensor ermöglicht es, benutzerspezifische Informationen für das Verfahren zu berücksichtigen, beispielsweise die Abmessungen des Körpers des Benutzers, oder bestimmte Eigenschaften dieses Körpers. Damit eine auf das Individuum abgestimmte Bestrahlung möglich. Die individuellen Einstellparameter des Benutzers können vorzugsweise auf einem Speicher der Vorrichtung oder des Benutzers gespeichert und bei Bedarf eingelesen werden.

Gemäß einer ersten bevorzugten Ausgestaltung des Verfahrens ist vorgesehen, dass der Benutzersensor die Eigenschaften des Körpers, ausgewählt aus der Gruppe umfassend die Höhe, die Breite, den Umfang des Körpers, ermittelt und die Lage und/oder die Intensität der Bestrahlungsmodule an die erfassten Eigenschaften angepasst wird. Die Lage der Bestrahlungsmodule kann durch Zustellung in Richtung auf den Körper verändern werden, wobei Mittel zum Zustellen zweckmäßigerweise von der Steuerung entsprechend dem vorgegebenen Abstand zu dem Körper verlagert werden. Die Intensität der Bestrahlungsmodule kann durch die Steuerung eingestellt werden, wobei insbesondere bei einer Ausgestaltung der Bestrahlungsmodule als Träger mit einer Mehrzahl von punktuellen LEDs eine besonders effektive Anpassung an die Körpereigenschaften erreicht wird.

Gemäß einer weiteren bevorzugten Ausgestaltung ist vorgesehen, dass der Benutzersensor die Lage und/oder die Art von Hautmerkmalen des Körpers des Benutzers, ausgewählt aus der Gruppe umfassend Tätowierungen, Verbrennungen, Wunden, Muttermalte, Narben, weiße Flecken, Pigmentstörungen, Bräunung, Hauttyp erfasst werden, und die Lage und/oder die Intensität der Bestrahlungsmodule an die erfassten Hautmerkmale angepasst wird. So ist es beispielsweise möglich, dass bei Tätowierungen oder Muttermale gegebene erhöhte Absorbtionsverhalten hinsichtlich der medizinisch-kosmetischen Strahlung dadurch auszugleichen, dass die Strahlungsintensität der Bestrahlungsmodule bzw. der darin vorzugsweise vorgesehenen LEDs reduziert wird. In gleicher Weise können Hautstellen, die nicht in der Lage sind, eine Bräunung durch Pigmentierung zu erreichen, insbesondere auf Grund von Narben, weißen Flecken, Pigmentstörungen oder Wunden, ebenfalls mit reduzierter Leistung bestrahlt werden, um eine Schädigung der Haut oder der Unterhaut zuverlässig zu vermeiden. Schließlich kann der Benutzersensor auch den Hauttyp und die Bräunung, insbesondere die Pigmentierung der Haut erfassen und auf Grund der erfassten Hautmerkmale die Bestrahlungsmodule einstellen. Es ist möglich, wenn der Benutzersensor mehrfach die Haut des Körpers des Benutzers erfasst, auch den Hauttyp und die etwaige Bräunung des Benutzers zu ermitteln.

Vorzugsweise weisen die Bestrahlungsmodule LEDs auf, die zumindest zeitweise gepulst betrieben werden. Durch das gepulste Betreiben des LEDs ist es möglich, photobiologische Effekte des Körpers des Benutzers zu stimulieren.

Die vorstehend dargelegten Verfahren können insbesondere auch als Verfahren zum Betreiben der Vorrichtung, insbesondere der vorstehend dargelegten Vorrichtung, zum Bestrahlen ausgebildet sein, so dass sich ein Verfahren zum Betreiben einer Vorrichtung zum Bestrahlen ergibt, das die Merkmale des vorgenannten Verfahren aufweist.

Weitere Vorteile, Merkmale, Eigenschaften und Weiterbildungen der Erfindung ergeben sich aus der nachfolgenden Beschreibung eines bevorzugten Ausführungsbeispiels sowie aus den abhängigen Ansprüchen.

Die Erfindung wird nachstehend unter Bezugnahme auf die anliegenden Zeichnungen und anhand eines bevorzugten Ausführungsbeispiels näher erläutert.
- Fig. 1: zeigt eine schematische Seitenansicht eines bevorzugten Ausführungsbeispiels einer erfindungsgenmäßen Vorrichtung zum Bestrahlen.
- Fig. 2: zeigt eine explodierte Darstellung eines erfindungsgemäßen Bestrahlungsmoduls, das in der Vorrichtung aus Fig. 1 eingebaut ist.

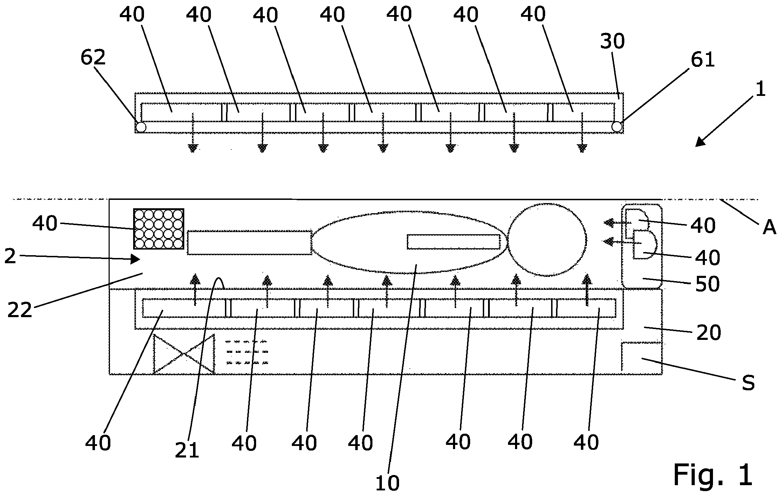

Fig. 1 zeigt eine Vorrichtung zum Bestrahlen eines mit ihrem menschlichen Körper 10 schematisch dargestellten Benutzers mit medizinisch-kosmetischer Strahlung, die ein unteres Gehäuseteil 20 und ein oberes Gehäuseteil 30 umfasst, die entlang einer Achse A gelenkig miteinander verbunden sind. Das obere Gehäuseteil 30 kann nach oben geschwenkt werden, um den Zugang für den Benutzer 10 frei zu geben, und kann herabgeschwenkt werden, damit die Gehäuseteile 20, 30 eine tunnelartige Röhre 2 umschließen, in der der Benutzer 10 liegt.

Die Gehäuseteile 20, 30 sind in Acrylglas gekapselt, wobei bei dem unteren Gehäuseteil 20 eine Liegefläche 21 aus Acrylglas ausgebildet ist. Es ist möglich, die Liegefläche mit einer Silikonmatte, die an die Liegefläche 10 angeschlossen ist, auszustatten, die nachgiebig ist und für die Person 10 eine angenehmere Haptik bereitstellt. Das Acrylglas und die Silikonmatte sind jeweils für zumindest Teile der medizinisch-kosmetischen Strahlung durchlässig.

In dem unteren Gehäuseteil 20 und dem oberen Gehäuseteil 30 sind jeweils Bestrahlungsmodule 40 angeordnet, die in den Gehäuseteilen 20, 30 in Richtung auf die Röhre 2 gerichtet sind. Die Bestrahlungsmodule 40 sind rechteckig aufgebaut und in dem unteren Gehäuseteil 20 nebeneinander liegend parallel zu der Liegefläche 21 angeordnet. Weiterhin sind an dem zu der Liegefläche 21 in etwa senkrechten vertikalen Abschnitt 22 des unteren Gehäuseteil 20 ebenfalls Bestrahlungsmodule 40 vorgesehen, die die Person 10 bestrahlen können. In dem oberen Gehäuseteil 30 sind in einer Reihe eine Mehrzahl von Bestrahlungsmodulen 40 jeweils aneinander stoßend angeordnet, wobei die in einer Reihe angeordneten Bestrahlungsmodule gegenüber der benachbarten Reihe um einen Winkel abgewinkelt sind, um die halbrunde Kontur des oberen Gehäuseteils 30 innerhalb des Gehäuseteils 30 nachvollziehen zu können. Der Winkel beträgt je nach Radius und je nach Größe der Bestrahlungsmodule zwischen 5° und 25°, vorzugsweise etwa 10°.

An einem Kopfende der Röhre 2 ist eine Schulterbräuneinrichtung 50 angeordnet, die insbesondere den Kopf und die Schulter des Benutzers 10 bestrahlt, wobei innerhalb der Schulterbräuneinrichtung 50 zwei weitere Bestrahlungsmodule 40 angeordnet sind.

Die Bestrahlungsmodule 40 sind mit einer Steuerung S der Vorrichtung 1 verbunden.

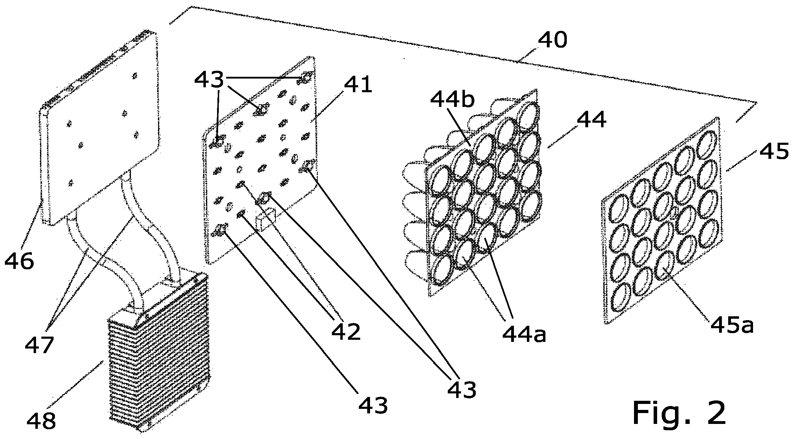

In Fig. 2 ist eine Explosionsdarstellung eines Steuerungsmoduls 40 veranschaulicht. Man erkennt, dass auf einem Träger 41 eine Mehrzahl von insgesamt zwanzig LEDs 42, 43 angebracht sind, die über den Träger 41 kontaktiert sind mit einer elektrischen Energieversorgung. Es ist möglich, die LEDs auch in einer anderen Zahl und/oder Anordnung als wie vorliegend in 4x5 Feldanordnung vorzusehen.

Man erkennt, dass insgesamt sechs LEDs 43 auf den Träger 41 angeordnet sind, die Strahlung des Spektrums UV-B emittieren, während die übrigen vierzehn LEDs 42 Strahlung des Spektrums UV-A emittieren.

Dem Träger 41 mit den LEDs 42, 43 vorgelagert ist eine Baueinheit 44 mit 20 identisch ausgebildeten Reflektoren 44a vorgesehen, wobei das Lochmaß der Reflektoren 44a auf die LEDs 42, 43 abgestimmt ist. Die Reflektoren 44a sind hierzu in einem von den LEDs 42, 43 beabstandeten Bereich mit einer Scheibe 44b, die Durchbrechungen für die Reflektoren 44a aufweist, verbunden, sodass die Baueinheit 44 wie ein Teil gehandhabt werden kann.

Der Baueinheit 44 vorgelagert ist eine Ringscheibe 45, die eine der Anzahl der Reflektoren 44a entsprechende Anzahl von kreisförmigen Ausnehmungen 45a in einem Plattenkörper aufweist, die in ihrem Innenumfang mit einem fluoreszierenden Material bestrichen sind. Werden die LEDs 42, 43 zum Emittieren der Strahlung veranlasst, regt diese Strahlung das fluoreszierende Material der Ringe 45a an, und auf Grund des im sichtbaren Bereich stattfindenden Leuchtens der Ringe 45a kann erkannt werden, dass die LEDs 42, 43 auch Strahlung emittieren.

An der den LEDs 42, 43 abgekehrten Seite des Trägers 41 ist eine Wärmeübergangsplatte 46 angeordnet, die als Plattenkörper ausgebildet ist und zur Ableitung der beim Betrieb des LEDs 42, 43 entstehenden Wärme bestimmt ist. Hierzu ist die Wärmeübergangsplatte 46 über eine erste Kühlleitung 47 und eine zweite Kühlleitung 47 mit einem als Wärmetauscher ausgebildeten Kühlkörper 48 verbunden, wobei zwischen der mit Hohlräumen ausgebildeten Wärmeübergangsplatte 46, der ersten Kühlleitung 47, den Kühlkörper 48 und der zweiten Kühlleitung 47 ein zirkulierendes Kühlfluid vorgesehen ist. Die Kühlung der Wärmeübergangsplatte 46 kann insbesondere durch Phasenumwandlung des Kühlfluids zwischen der Wärmeübergangsplatte 46 einerseits und dem Kühlkörper 48 andererseits bewirkt werden.

Die in dem Gehäuseteil 20 bzw. Gehäuseteil 30 verbauten Bestrahlungsmodule 40 sind alle gleichartig aufgebaut, es versteht sich aber, dass die Bestrahlungsmodule auch in Abhängigkeit von der Lichtempfindlichkeit bestimmter Partien des Benutzers 10 unterschiedlich aufgebaut und/oder angesteuert sein können.

In dem oberen Gehäuseteil 30 ist ein erster Sensor 61 vorgesehen, der die Eigenschaften des Körpers des Benutzers 10, insbesondere dessen Höhe, Breite, Umfang sowie die Lage der Arme, Beine ermittelt. Je nach erfassten Eigenschaften des Körpers werden die Bestrahlungsmodule 40 in ihrer Strahlung eingestellt. So kann beispielsweise das Bestrahlungsmodul 40, das dem Kopfende abgekehrt ist, ganz abgeschaltet sein, wenn die Beine des Benutzers dieses Bestrahlungsmodul 40 nicht mehr überdecken.

Der Sensor 61 kann alternativ oder zusätzlich bestimmte Hautmerkmale des Körpers des Benutzers 10 erfassen, beispielsweise das Vorliegen von Tätowierungen, von Verbrennungen, von Wunden, von Muttermalen, von Narben, von weißen Flecken, von Pigmentstörungen, der aktuelle Bräunung und auch des Hauttyps. Dieser zweite Sensor, der auch als Kamera ausgebildet sein und an den ein Auswertelogik angeschlossen ist, erfasst Färbung und Kontraste der Haut mit einer großen Auflösung und wertet die erfassten Bilder aus, um die genannten Besonderheiten des Körpers zu bestimmen. In Abhängigkeit von den Hautmerkmalen wird dann das Bestrahlungsmodul 40 mit einer reduzierten Leistung betrieben, wenn auf Grund der detektierten Hautmerkmale die Verbrennung der Haut bei normaler Strahlung und Exposition zu befürchten ist.

Schließlich ist in dem oberem Gehäuseteil noch ein zweiter Sensor 62 angeordnet, der die Strahlung der Bestrahlungsmodule 40 bzw. der zugehörigen LEDs 42, 43 detektiert. Der zweite Sensor 62 bzw. dessen Auswerteeinheit vergleicht die erfasste Strahlung mit z.B. in der Steuerung S gespeicherten SollWerten, und in Reaktion auf eine Abweichung der detektierten Werte von den SollWerten bewirkt die Steuerung, dass die Betriebsparameter der Bestrahlungsmodule 40 so verstellt werden, dass sich ein Einregeln auf den SollWert ergibt.

Die Erfindung ist vorstehend anhand eines Ausführungsbeispiels erläutert worden, bei dem das Bestrahlungsmodul mit zwei Typen von LEDs 42, 43 ausgestattet ist, die unterschiedliche ultraviolette Spektren aussenden. Es versteht sich, dass weitere LEDs mit einem von den beiden LEDs 42, 43 verschiedenen Spektrum ebenfalls in dem Bestrahlungsmodul vorgesehen sein können.

Die Erfindung ist vorstehend anhand eines Ausführungsbeispiels erläutert worden, bei dem das Bestrahlungsmodul sechs LEDs 43 mit einem UV-B Spektrum und vierzehn LEDs mit einem UV-A Spektrum aufweist. Es versteht sich, dass die Anzahl der LEDs mit dem entsprechenden Spektrum je nach Anwendungsfall auch anders aufgeteilt sein kann.

Die Erfindung ist vorstehend anhand eines Ausführungsbeispiels erläutert worden, bei dem der Träger 41 des Bestrahlungsmoduls 40 im Wesentlichen rechteckig ausgebildet ist und ein Feld von 4x5 LEDs 42, 43 aufweist. Es versteht sich, dass der Träger 41 auch eine andere Gestalt, beispielsweise quadratisch oder hexagonal aufweisen kann, und dass die LEDs 42, 43 auch anders auf dem Träger 41 angeordnet sein können.

Die Erfindung ist vorstehend anhand eines Ausführungsbeispiels erläutert worden, bei dem der Träger 41 auf einer Wärmeübergangsplatte 46 angeschlossen ist, die über Kühlleitungen 47 mit einem Wärmetauscher verbunden ist. Es versteht sich, dass der Wärmetauscher 48 über weitere Kühlleitungen zugleich mit einem weiteren Träger 41 verbunden sein kann, und dass es auch möglich ist, mehrere Wärmeübergangsplatten über Verbindungsleitungen zu einem geschlossenen System mit dem Wärmetauscher 48 zu verbinden.

Die Erfindung ist vorstehend anhand eines Ausführungsbeispiels erläutert worden, bei dem sämtliche Bestrahlungsmodule 40 in der Vorrichtung 1 in gleicher Weise ausgebildet sind. Es versteht sich, dass die Bestrahlungsmodule 40 für den Schulter- und Kopfbereich, die Bestrahlungsmodule in dem unteren Gehäuseteil 20 und die Bestrahlungsmodule in dem oberen Gehäuseteil 30 jeweils auch verschieden ausgebildet sein können, und insbesondere auch eine unterschiedliche Anzahl von LEDs aufweisen können.

Die Erfindung ist vorstehend anhand eines Ausführungsbeispiels erläutert worden, bei dem die Bestrahlungsmodule 40 ortsfest in den Gehäuseteilen 20, 30 angeordnet sind, und im Wesentlichen in Reaktion von den ersten Sensor 61 und zweiten Sensor 62 erfassten Daten angesteuert werden. Es versteht sich, dass statt einer elektrischen Ansteuerung der Bestrahlungsmodule 40 diese auch hinsichtlich ihrer Entfernung zu dem Körper des Benutzers 10 einstellbar sein können, beispielsweise über pneumatische, hydraulische, mechanische oder elektrische Verstelleinrichtungen.

Die Erfindung ist vorstehend anhand eines Ausführungsbeispiels erläutert worden, bei dem die Vorrichtung 1 ein stationäres Unterteil 20 und ein schwenkbar auf das Unterteil 20 herabschwenkbares oberes Gehäuseteil 30 aufweist, wobei der Benutzer 10 auf einer Liegefläche 21 des unteren Gehäuseteils 20 ruht. Es versteht sich, dass die Vorrichtung auch in der Gestalt eines Stehbräuners ausgebildet sein kann, bei dem die beiden Gehäuseteile im Wesentlichen senkrecht stehend angeordnet sind, und bei dem der Benutzer während der Bestrahlung im Wesentlichen auf dem Boden steht und von dem Gehäuseteilen umgeben ist.

Die Erfindung ist vorstehend anhand eines Ausführungsbeispiels erläutert worden, bei dem ein Sensor 61, 62 Eigenschaften der Vorrichtung 1 bzw. der Person 10 erfasst. Es versteht sich, dass hierfür auch mehrere Sensoren vorgesehen sein können, und dass die von den Sensoren gewonnenen Daten auch gespeichert werden können, um die ordnungsgemäße Einstellung der Vorrichtung zu dokumentieren.

## Patentansprüche

1. Bestrahlungsmodul zum Einsatz in einer Vorrichtung (1) zum Bestrahlen mit medizinisch-kosmetischer Strahlung, umfassend
eine Mehrzahl von auf einem Träger (41) angeordneter LEDs (42; 43), wobei
auf dem Träger (41) eine erste Gruppe von ersten LEDs (42), die Strahlung des Spektrums UV-A emittiert, und eine zweite Gruppe von zweiten LEDs (43), die Strahlung des Spektrums UV-B emittiert, angeordnet sind, und die ersten LEDs zwischen 50% und 90% der Summe aus ersten und zweiten LEDs ausmachen, und dass die zweiten LEDs (43) zwischen 50% und 10% der Summe aus ersten und zweiten LEDs ausmachen,
**dadurch gekennzeichnet,**
**dass** der Träger an eine Wärmeübergangsplatte (46) angeschlossen ist, und dass jeder LED (42; 43) ein Reflektor (44a) zugeordnet ist, der an den Träger (41) angeschlossen ist, dass die sich kegelig von der LED (42; 43) fort erweiternden Reflektoren (44a) als Feld in einer gemeinsamen Baueinheit (44) angeordnet sind, und dass die Baueinheit (44), der Träger (41) und die Wärmeübergangsplatte (46) miteinander verbunden sind.

2. Bestrahlungsmodul nach Anspruch 1, wobei auf einem Träger zwanzig LEDs (42; 43) oder ein ganzzahliges Vielfaches davon angeordnet sind, und jeweils vier oder ein ganzzahliges Vielfaches davon der LEDs als zweite LEDs (43) ausgebildet sind.

3. Bestrahlungsmodul nach einem der vorhergehenden Ansprüche, wobei die LEDs (42; 43) auf dem Träger in einem Feld aus vier Zeilen und fünf Spalten angeordnet sind, und die mittlere Spalte durch zweite LEDs (43) ausgebildet ist.

4. Bestrahlungsmodul nach Anspruch 3, wobei der Abstand benachbarter LEDs (42; 43) in derselben Spalte oder Zeile zwischen 1 cm und 4 cm, vorzugsweise zwischen 1,25 cm und 2,25 cm und besonders bevorzugt zwischen 1,5 cm und 2 cm beträgt.

5. Bestrahlungsmodul nach einem der vorhergehenden Ansprüche, wobei die LEDs (42; 43) mit einer Primäroptik in Gestalt einer Silikon-Linse ausgestattet sind.

6. Bestrahlungsmodul nach einem der vorhergehenden Ansprüche, wobei dass die zweiten LEDs (43) mit einer geringeren Leistung angesteuert werden als die ersten LEDs (42).

7. Bestrahlungsmodul nach einem der vorhergehenden Ansprüche, wobei die Wärmeübergangsplatte (46) über Kühlleitungen (47) mit einem Wärmetauscher (48) verbunden ist.

8. Bestrahlungsmodul nach einem der vorhergehenden Ansprüche, wobei die Baueinheit (44), der Träger (41) und die Wärmeübergangsplatte (46) miteinander verschraubt sind.

9. Bestrahlungsmodul nach Anspruch 9, wobei die Baueinheit (44) wenigstens eine Aussparung für eine ohne Reflektor ausgestattete dritte LED aufweist, die sichtbare Strahlung emittiert.

10. Vorrichtung zum Bestrahlen eines Benutzers mit medizinisch-kosmetischer Strahlung, umfassend wenigstens ein Bestrahlungsmodul (40) nach einem der vorhergehenden Ansprüche.

11. Vorrichtung nach Anspruch 10, wobei eine Eingabeeinheit für den Benutzer (10) vorgesehen ist, und der Benutzer (10) Bereiche auswählen kann, in denen die dem ausgewählten Bereich zugeordneten LEDs (42; 43) mit verminderter Leistung oder nicht angesteuert werden.

12. Vorrichtung nach Anspruch 10, wobei zum Erfassen eines zu bestrahlenden Benutzers (10) ein Sensor (61) vorgesehen ist, der den Körper des Benutzers erfasst.

13. Vorrichtung zum Bestrahlen eines Benutzers (10) mit medizinisch-kosmetischer Strahlung nach Anspruch 10, umfassend
eine Mehrzahl von Bestrahlungsmodulen (40) zum Bestrahlen mit medizinisch-kosmetischer Strahlung nach einem der Ansprüche 1-9, die in einem Gehäuseteil (20; 30) untergebracht sind,
wobei ein Sensor (62) vorgesehen ist, der die von den Bestrahlungsmodulen (40) emittierte Strahlung erfasst, und
die Bestrahlungsmodule (40) in Reaktion auf eine Abweichung der erfassten Strahlung von einem vorgebbaren Wert der Strahlung von einer Steuerung (S) mit veränderten Betriebsparametern ansteuerbar sind, um die emittierte Strahlung an den vorgebbaren Wert anzupassen.

14. Vorrichtung nach Anspruch 13, wobei die Bestrahlungsmodule (40) mit konstanter Spannung betrieben werden, und die Ansteuerung der Bestrahlungsmodule (40) mittels Pulsweitenmodulation erfolgt.

15. Vorrichtung nach Anspruch 13, wobei die Bestrahlungsmodule (40) mit konstantem Strom betrieben werden, und die Ansteuerung der Bestrahlungsmodule (40) mittels Veränderung der Stromstärke erfolgt.

## Claims

1. An irradiation module for use in a device (1) for irradiation with medical-cosmetic radiation, comprising
a plurality of LEDs (42; 43) arranged on a carrier (41),
a first group of first LEDs (42), which emit radiation in the UVA spectrum, and a second group of second LEDs (43), which emit radiation in the UVB spectrum, being arranged on the carrier (41), and the first LEDs making up between 50% and 90% of the total from the first and second LEDs, and the second LEDs (43) making up between 50% and 10% of the total from the first and second LEDs, **characterized in that**
the carrier is connected to a heat-transfer plate (46), and **in that** a reflector (44a) is assigned to each LED (42; 43), which reflector is connected to the carrier (41), **in that** the reflectors (44a) extending from the LEDs (42; 43) in a tapering manner are arranged as a field in a common modular unit (44), and **in that** the modular unit (44), the carrier (41), and the heat-transfer plate (46) are interconnected.

2. The irradiation module according to claim 1, wherein twenty LEDs (42; 43) or an integer multiple thereof are arranged on a carrier, and four or an integer multiple of the LEDs are each formed as second LEDs (43).

3. The irradiation module according to any of the preceding claims, wherein the LEDs (42; 43) are arranged on the carrier in a field made up of four rows and five columns, and the middle column is formed by second LEDs (43).

4. The irradiation module according to claim 3, wherein the distance between adjacent LEDs (42; 43) in the same column or row is between 1 cm and 4 cm, preferably between 1.25 cm and 2.25 cm, and particularly preferably between 1.5 cm and 2 cm.

5. The irradiation module according to any of the preceding claims, wherein the LEDs (42; 43) are equipped with primary optics in the form of a silicone lens.

6. The irradiation module according to any of the preceding claims, wherein the second LEDs (43) are actuated at a lower power level than the first LEDs (42).

7. The irradiation module according to any of the preceding claims, wherein the heat-transfer plate (46) is connected to a heat exchanger (48) via cooling lines (47).

8. The irradiation module according to any of the preceding claims, wherein the modular unit (44), the carrier (41), and the heat-transfer plate (46) are screwed together.

9. The irradiation module according to claim 9, wherein the modular unit (44) comprises at least one recess for a third LED that is not equipped with a reflector and emits visible radiation.

10. A device for irradiation of a user with medical-cosmetic radiation, comprising at least one irradiation module (40) according to any of the preceding claims.

11. The device according to claim 10, wherein an input unit is provided for the user (10), and the user (10) can select regions in which the LEDs (42; 43) assigned to the selected region are actuated at reduced power or are not actuated at all.

12. The device according to claim 10, wherein a sensor (61), which detects the body of the user, is provided for detecting a user (10) to be irradiated.

13. A device for irradiation of a user (10) with medical-cosmetic radiation according to claim 10, comprising
a plurality of irradiation modules (40) for irradiation with medical-cosmetic radiation according to any of claims 1-9, which modules are housed in a housing part (20; 30), wherein a sensor (62) is provided which detects the radiation being emitted by the irradiation modules (40), and
the irradiation modules (40) can be actuated by a controller (S) with modified operating parameters in response to a deviation in the detected radiation from a specifiable value for the radiation, in order to adjust the emitted radiation to the specifiable value.

14. The device according to claim 13, wherein the irradiation modules (40) are operated at a constant voltage, and the irradiation modules (40) are actuated by means of pulse-width modulation.

15. The device according to claim 13, wherein the irradiation modules (40) are operated at a constant current, and the irradiation modules (40) are actuated by means of modifying the amperage.

## Revendications

1. Module d'irradiation destiné à être utilisé dans un dispositif (1) d'irradiation avec un rayonnement médico-cosmétique, comprenant
une pluralité de LED (42 ; 43) disposées sur un support (41), dans lequel
un premier groupe de premières LED (42) qui émet le rayonnement du spectre UV-A et un deuxième groupe de deuxièmes LED (43) qui émet le rayonnement du spectre UV-B sont disposés sur le support (41), et les premières LED représentent entre 50 % et 90 % de la somme des premières et deuxièmes LED, et que les deuxièmes LED (43) représentent entre 50 % et 10 % de la somme des premières et deuxièmes LED,
**caractérisé en ce que** le support est connecté à un plateau de transfert thermique (46), et **en ce qu'**à chaque LED (42 ; 43) est associé un réflecteur (44a) qui est connecté au support (41), **en ce que** les réflecteurs (44a) s'élargissant de manière conique en s'éloignant des LED (42 ; 43) sont disposés sous la forme d'une matrice dans une unité modulaire (44) commune, et **en ce que** l'unité modulaire (44), le support (41) et le plateau de transfert thermique (46) sont reliés les uns aux autres.

2. Module d'irradiation selon la revendication 1, dans lequel vingt LED (42 ; 43) ou un multiple entier de vingt sont disposées sur un support, et respectivement quatre ou un multiple entier de quatre des LED sont réalisées en tant que deuxièmes LED (43).

3. Module d'irradiation selon l'une quelconque des revendications précédentes, dans lequel les LED (42 ; 43) sur le support sont disposées dans une matrice de quatre lignes et de cinq colonnes, et la colonne centrale est réalisée par des deuxièmes LED (43).

4. Module d'irradiation selon la revendication 3, dans lequel la distance de LED voisines (42 ; 43) dans la même colonne ou la même ligne est comprise entre 1 cm et 4 cm, de préférence entre 1,25 cm et 2,25 cm, et de manière particulièrement préférée entre 1,5 cm et 2 cm.

5. Module d'irradiation selon l'une quelconque des revendications précédentes, dans lequel les LED (42 ; 43) sont équipées d'une optique primaire sous la forme d'une lentille de silicone.

6. Module d'irradiation selon l'une quelconque des revendications précédentes, dans lequel les deuxièmes LED (43) sont pilotées avec une puissance inférieure à celle des premières LED (42).

7. Module d'irradiation selon l'une quelconque des revendications précédentes, dans lequel le plateau de transfert thermique (46) est relié à un échangeur thermique (48) par des conduites de refroidissement (47).

8. Module d'irradiation selon l'une quelconque des revendications précédentes, dans lequel l'unité modulaire (44), le support (41) et le plateau de transfert thermique (46) sont vissés les uns aux autres.

9. Module d'irradiation selon la revendication 9, dans lequel l'unité modulaire (44) présente au moins un évidement pour une troisième LED sans réflecteur qui émet un rayonnement visible.

10. Dispositif permettant d'irradier un utilisateur avec un rayonnement médico-cosmétique, comprenant au moins un module d'irradiation (40) selon l'une quelconque des revendications précédentes.

11. Dispositif selon la revendication 10, dans lequel une unité d'entrée est prévue pour l'utilisateur (10), et l'utilisateur (10) peut sélectionner des zones dans lesquelles les LED (42 ; 43) associées à la zone sélectionnée peuvent être pilotées avec une puissance réduite ou ne pas être pilotées du tout.

12. Dispositif selon la revendication 10, dans lequel un capteur (61) est prévu pour détecter un utilisateur (10) à irradier et qui détecte le corps de l'utilisateur.

13. Dispositif permettant d'irradier un utilisateur (10) avec un rayonnement médico-cosmétique selon la revendication 10, comprenant
une pluralité de modules d'irradiation (40) pour une irradiation avec un rayonnement médico-cosmétique selon l'une quelconque des revendications 1 à 9 qui sont logés dans une partie de boîtier (20 ; 30),
dans lequel un capteur (62) est prévu qui détecte le rayonnement émis par les modules d'irradiation (40), et
en réponse à un écart du rayonnement détecté par rapport à une valeur prédéfinissable du rayonnement, les modules d'irradiation (40) peuvent être pilotés par une unité de commande (S) avec des paramètres opérationnels modifiés afin d'adapter le rayonnement émis à la valeur prédéfinissable.

14. Dispositif selon la revendication 13, dans lequel les modules d'irradiation (40) fonctionnent à tension constante, et le pilotage des modules d'irradiation (40) est effectué au moyen d'une modulation de largeur d'impulsion.

15. Dispositif selon la revendication 13, dans lequel les modules d'irradiation (40) fonctionnent avec du courant constant, et le pilotage des modules d'irradiation (40) est effectué au moyen d'une variation de l'intensité de courant.
